Europäisches Patentamt

European Patent Office

Office européen des brevets

(19)

(11) Veröffentlichungsnummer : **0 084 648**
**B1**

(12) EUROPÄISCHE PATENTSCHRIFT

(45) Veröffentlichungstag der Patentschrift :
07.11.84

(21) Anmeldenummer : 82111632.4

(22) Anmeldetag : 15.12.82

(51) Int. Cl.³ : **C 07 C 21/24, C 07 C 17/00**

(54) Verfahren zur Isomerisierung von Alkylbenzotrifluoriden.

(30) Priorität : 24.12.81 DE 3151364

(43) Veröffentlichungstag der Anmeldung :
03.08.83 Patentblatt 83/31

(45) Bekanntmachung des Hinweises auf die Patenterteilung : 07.11.84 Patentblatt 84/45

(84) Benannte Vertragsstaaten :
BE CH DE FR GB IT LI

(56) Entgegenhaltungen :
US-A- 3 560 579
US-A- 3 577 470

(73) Patentinhaber : BAYER AG
Konzernverwaltung RP Patentabteilung
D-5090 Leverkusen 1 Bayerwerk (DE)

(72) Erfinder : Marhold, Albrecht, Dr.
Carl-Duisberg-Strasse 329
D-5090 Leverkusen 1 (DE)
Erfinder : Klauke, Erich, Dr.
Eichendorffweg 8
D-5068 Odenthal (DE)

**Beschreibung**

Die Erfindung betrifft ein Verfahren zur Isomerisierung und nachfolgender Isolierung von Alkylbenzotrifluoriden.

Bei der Herstellung von Alkylbenzotrifluoride durch Alkylierung von Benzotrifluorid entsteht im allgemeinen ein Isomerengemisch, das hauptsächlich aus den para- und meta-Isomeren besteht. Für die Weiterverarbeitung der Benzotrifluoride, beispielsweise zu Trifluormethylbenzylchloriden (US 3 465 051) ist es in der Regel erforderlich, die reinen Isomeren einzusetzen. Diese kann man erhalten, wenn man das Isomerengemisch destilliert. Bei dieser Verfahrensweise fällt immer ein unerwünschter Anteil des nicht benötigten Isomeren als Nebenprodukt an, das sich nicht zu dem gewünschten Endprodukt verarbeiten läßt.

Aufgabe der Erfindung ist es daher, aus einem in an sich bekannter Weise hergestellten Isomerengemisch aus Alkylbenzotrifluoriden praktisch ausschließlich ein Isomeres zu isolieren, wobei kein Verlust und kein Zwangsanfall an dem anderen Isomeren auftritt.

Hierzu wurde ein Verfahren zur Isomerisierung von Benzotrifluoriden, die durch Alkylgruppen substituiert sind, gefunden, das dadurch gekennzeichnet ist, daß man die Alkylbenzoltrifluoride mit Fluorwasserstoff behandelt.

Die durch Alkylgruppen substituierten Benzotrifluoride können gegebenenfalls durch vorzugsweise einen Fluor-, Chlor- oder Brom-Rest substituiert sein.

Selbstverständlich ist es nach dem erfindungsgemäßen Verfahren auch möglich, außer Isomerengemischen auch eine reines Isomer einzusetzen und es in ein anderes umzuwandeln.

Nach dem erfindungsgemäßen Verfahren erhält man vorzugsweise als meta- und para-Isomere ein Monoalkylbenzotrifluorid bzw. das syn- und asyn-Isomere eines Dialkylbenzotrifluorids.

Alkylreste der Alkylbenzotrifluoride sind vorzugsweise niedere, geradkettige oder verzweigte Kohlenwasserstoffreste (Niederalkyl) mit 1 bis etwa 6 Kohlenstoffatomen. Beispielsweise seien Methyl, Ethyl, Propyl, Isopropyl, Butyl, Isobutyl, Pentyl, Isopentyl, Hexyl und Isohexyl genannt.

Alkylbenzotrifluoride gemäß der vorliegenden Erfindung enthalten eine oder mehrere Alkylgruppen. Bevorzugt werden jedoch für das erfindungsgemäße Verfahren Mono- und Dialkylbenzotrifluoride eingesetzt.

Der für die erfindungsgemäße Isomerisierung günstigste Temperaturbereich ist abhängig von der speziellen Verbindung und dem angestrebten Isomeren. Im allgemeinen führt man die Isomerisierung im Temperaturbereich von 0 bis 200 °C bevorzugt von 10 bis 150 °C, durch.

Durch die Wahl einer niederen Temperatur begünstigt man im allgemeinen die Bildung des para- bzw. asym-Isomeren, die höhere Temperatur begünstigt die Bildung des meta- bzw. sym-Isomeren.

Die Isomerisierung von Methylbenzotrifluorid führt man beispielsweise bevorzugt im Temperaturbereich von 100 bis 150 °C durch.

Bei einer Durchführung der erfindungsgemäßen Isomerisierung bei höheren Temperaturen führt man das Verfahren zweckmäßigerweise unter Druck, bevorzugt in einem Autoklaven unter dem Eingendruck des Systems, durch. Im allgemeinen führt man das erfindungsgemäße Verfahren bei einem Druck im Bereich von etwa 1 bis 150 bar durch.

Auch durch die Wahl des Druckes läßt sich die Lage des Isomeren-Gleichgewichts verändern. Im allgemeinen begünstigt ein höherer Druck die Bildung des meta- bzw. sym-Isomeren und ein niederer Druck die Bildung des para- bzw. asym-Isomeren.

Bei dem Arbeiten unter Druck erhöht sich die Löslichkeit des Fluorwasserstoffs in dem Alkylbenzotrifluorid und damit auch die Isomerisierungsgeschwindigkeit.

Die Menge des zur Isomerisierung eingesetzten Fluorwasserstoffs kann innerhalb eines sehr großen Bereichs liegen. Da durch die geringe Löslichkeit des Alkylbenzotrifluorids in Fluorwasserstoff bedingt das erfindungsgemäße Verfahren im allgemeinen in einem Zweiphasensystem durchgeführt wird, wird die Isomerisierungsgeschwindigkeit u. a. durch den Grad der Vermischung beider Phasen bestimmt. Um eine für technische Zwecke befriedigende Isomerisierungsgeschwindigkeit zu erzielen, ist daher eine Mindestmenge an Fluorwasserstoff zweckmäßig. Diese zweckmäßige Mindestmenge hängt weitgehend von den apparativen Gegebenheiten ab.

Die Obergrenze der Fluorwasserstoffmenge hängt weitgehend nur von der wirtschaflichen Zweckmäßigkeit ab und ist für das erfindungsgemäße Verfahren nicht begrenzt.

Im allgemeinen wird man das erfindungsgemäße Verfahren mit einer Menge von 0,5 bis 20 Mol, bevorzugt 2 bis 10 Mol, Fluorwasserstoff, bezogen auf 1 Mol Alkylbenzotrifluorid, durchführen.

Bei einem Überschuß an Fluorwasserstoff wird dieser als Lösungs- bzw. Verdünnungsmittel eingesetzt.

Es ist selbstverständlich auch möglich, das erfindungsgemäße Verfahren in Gegenwart anderer Lösungs- oder Verdünnungsmittel durchzuführen. Hierbei kommen Lösungs- oder Verdünnungsmittel in Frage, die sich unter den erfindungsgemäßen Bedingungen nicht verändern und gegebenenfalls die Löslichkeit der Komponenten ineinander erhöht. Beispielsweise seien die folgenden Lösungs- oder Verdünnungsmittel genannt: Trichlorfluormethan, 1,1,2-Trichlor-1,2,2-Trifluorethan, 1,1,1-Trifluor-2,2-dichlorethan, 1,3-Bistrifluormethylbenzol, 3-Nitrobenzotrifluorid.

Das erfindungsgemäße Verfahren kann auch in der Gasphase durchgeführt werden. Hierbei leitet man die Komponenten durch ein Reaktionsrohr, das gegebenenfalls Füllkörper enthält.

Es ist möglich, das erfindungsgemäße Verfahren diskontinuierlich oder kontinuierlich durchzuführen.

Beispielsweise führt man das erfindungsgemäße Verfahren wie folgt durch :

Die Reaktionskomponenten werden in beliebiger Reihenfolge gemischt und gegebenenfalls in Gegenwart eines Lösungs- bzw. Verdünnungsmittels und gegebenenfalls bei erhöhtem Druck auf die Isomerisierungstemperatur erwärmt. Die Einstellung des Gleichgewichts kann man mit Hilfe von analytischen Methoden, z. B. durch GC-Analyse, feststellen. Dann trennt man die Komponenten auf. Die nicht erwünschte Komponente wird man, soweit sie noch in größeren Mengen vorliegt, nochmals der erfindungsgemäßen Isomerisierung unterwerfen und so, gegebenenfalls nach mehrmaliger Wiederholung, nur praktisch ausschließlich ein Alkylbenzotrifluorid-Isomeres erhalten.

Die Trennung der Komponenten wird im allgemeinen durch Destillation durchgeführt. Hierbei wird zuerst der Fluorwasserstoff abgetrennt, der wieder bei einer weiteren Isomerisierung eingesetzt werden kann.

Insbesondere bei einer kontinuierlichen Arbeitsweise trennt man nur das gewünschte Isomere ab und führt den Rest in einem Kreisprozeß dem erfindungsgemäßen Verfahren wieder zu.

Es sind selbstverständlich zahlreiche Verfahren bekannt, alkylierte Aromaten zu isomerisieren (C.C. Price, Org. Reactions 3, 1-82 (1946) G.A. Olah, Friedel Crafts and Related Reactions Vol II, Interscience Publ. New Nork 1964).

Als Katalysatoren verwendet man hierbei Verbindungen, die eine Friedel-Crafts-Aktivität besitzen und auch für Alkylierungen von Aromaten eingesetzt werden. Nach diesen Verfahren lassen sich beispielsweise die isomeren Xylole ineinander umwandeln.

Diese Isomerisierungsmethode versagt jedoch, wenn neben der Alkylgruppe noch Substituenten vorhanden sind, die selbst Friedel-Crafts-Reaktionen eingehen können. Aus diesem Grund sind keine Methoden bekannt, mit denen man Alkylbenzotrifluoride isomerisieren kann. Die Trifluormethylgruppe in dem Benzotrifluorid reagiert nämlich mit den Friedel-Crafts-Katalysatoren unter Selbstkondensation zu harzartigen Produkten (J. Am. Chem. Soc. 1983, 60, 1967 und Bull. Soc. Chem. France 1962, 587). Bei den harzartigen Produkten ist keine Isomerisierung zu erwarten.

Die reinen Alkylbenzotrifluoridisomeren sind Zwischenprodukte für Aniline, die z. B. für Farbstoffe oder Pflanzenschutzmittel von Interesse sind (GB 2 027 699, DE-OS 2 750 170).

Beispiel 1

In einem VA-Autoklaven wurden 200 ml wasserfreier Fluorwasserstoff vorgelegt und 100 g einer Mischung aus 3-Isopropylbenzotrifluorid (78 %) und 4-Isopropyl-benzotrifluorid (22 %) zugegeben. Durch Aufbringung von Stickstoff wurde der Druck im Autoklaven auf 3 bar erhöht. Dann erhitzte man das Reaktionsgemisch 3 Stunden auf 60 °C. Nach dem Abkühlen und Abdestillieren des Fluorwasserstoffs wurde das Isomerenverhältnis der Isopropylbenzotrifluoride durch gaschromatographische Analyse bestimmt. Es liegen 91 Gew.-% 3-Isopropyl- und 9 % 4-Isopropylbenzotrifluorid vor.

Beispiel 2

In einem VA-Autoklaven wurde eine Mischung aus 100 ml Fluorwasserstoff und 80 g 3-Isopropylbenzotrifluorid vorgelegt. Der Druck im Autoklaven wurden durch Aufdrücken von Stickstoff auf 10 bar erhöht. Dann wurde das Reaktionsgemisch 6 Stunden bei 15 °C gerührt. Die gaschromatographische Analyse ergab eine Zusammensetzung von 21,2 % 4-Isopropylbenzotrifluorid und 78,8 Gew.-% 3-Isopropylbenzotrifluorid.

Beispiel 3

50 ml Fluorwasserstoff und 50 g 3,5-Diisopropylbenzotrifluorid wurden 5 Stunden bei 15 °C unter Normaldruck gerührt. Die gaschromatographische Analyse ergab einen Anteil von 3,5 % 2,5-Diisopropylbenzotrifluorid neben 96 % des Ausgangsmaterials.

Beispiel 4

Bei einer Wiederholung von Beispiel 3 unter einem Stickstoffdruck von 10 bar, stieg der Anteil an 2,5-Diisopropylbenzotrifluorid auf 9,1 % an.

Beispiel 5

Bei einer Wiederholung von Beispiel 4 bei einem Druck von 10 bar und einer Erhöhung des Verhältnisses Fluorwasserstoff zu 3,5-Diisopropylbenzotrifluorid auf 4 : 1 stieg der Anteil an 2,5-Diisopropylbenzotrifluorid auf 11,8 Gew.-% an.

Beispiel 6

Man rührt eine Mischung aus 30 ml Fluorwasserstoff und 50 g 2,5-Diisopropylbenzotrifluorid unter einem Stickstoffdruck von 8 bar für 3 Stunden bei 60 °C. Die gaschromatographische Analyse zeigt ein Verhältnis von 87,8 Gew.-% Ausgangsmaterial zu 12,2 Gew.-% 3,5-Diisopropylbenzotrifluorid an.

Beispiel 7

Eine Mischung aus 50 ml Fluorwasserstoff und 50 g 2-Methylbenzotrifluorid wurde für 5 Stunden auf 120 °C erhitzt, der Fluorwasserstoff abdestilliert und das organische Material mit Wasserdampf destilliert. Die gaschromatographi-

sche Analyse zeigte einen Anteil von 3,5 % 3-Methylbenzotrifluorid in 95,9 % 2-Methylbenzotrifluorid.

Beispiel 8

Man erhitzt eine Mischung aus 30 ml Fluorwasserstoff und 50 g 2-Methyl-5-brom-benzotrifluorid unter einem Stickstoffdruck von 28 bar für 5 Stunden auf 140 °C. Nach dem Abkühlen wurde die organische Phase analysiert. Durch die Isomerisierung hatte man 7,2 Gew.-% 3-Methyl-5-brombenzotrifluorid erhalten.

Beispiel 9

Man erhitzt eine Mischung von 50 ml Fluorwasserstoff und 18 g 3,4-Dimethylbenzotrifluorid für 5 Stunden auf 125 °C unter einem Stickstoffdruck von 25 bar. Die gaschromatographische Analyse zeigte nach der Destillation der Reaktionsmischung folgende Verteilung :

64,2 Gew.-% Ausgangsmaterial
24,9 Gew.-% 3,5-Dimethylbenzotrifluorid und
10,9 Gew.-% 2,5-Dimethylbenzotrifluorid.

Beispiel 10 (Vergleichsbeispiel)

Man legte 100 g 2-Methylbenzotrifluorid bei 20 °C vor und leitete 10 g Bortrifluorid ein. Es setzte augenblicklich eine Verharzung zu höhermolekularen Verbindungen ein. Es konnte hieraus kein Isomerisierungsprodukt isoliert werden.

**Ansprüche**

1. Verfahren zur Isomerisierung von Benzotrifluoriden, die durch eine Alkylgruppe und gegenbenenfalls durch einen Fluor-, Chlor- oder Brom-Rest substituiert sind, dadurch gekennzeichnet, daß man ein Isomerengemisch der Alkylbenzotrifluoride mit Fluorwasserstoff behandelt.

2. Verfahren nach Anspruch 1, dadurch gekennzeichnet, daß man die Umsetzung im Temperaturbereich von 0 bis 200 °C durchführt.

3. Verfahren nach den Ansprüchen 1 und 2, dadurch gekennzeichnet, daß man das Verfahren im Druckbereich von 1 bis 150 bar durchführt.

4. Verfahren nach den Ansprüchen 1 bis 3, dadurch gekennzeichnet, daß man 2 bis Mol Fluorwasserstoff, bezogen auf 1 Mol des Alkylbenzotrifluorids, einsetzt.

**Claims**

1. Process for the isomerisation of benzotrifluorides which are substituted by an alkyl group and optionally by a fluorine, chlorine or bromine radical, characterised in that a mixture of isomers of the alkylbenzotrifluorides is treated with hydrogen fluoride.

2. Process according to Claim 1, characterised in that the reaction is carried out in the temperature range of 0 to 200 °C.

3. Process according to Claims 1 and 2, characterised in that the process is carried out in the pressure range of 1 to 150 bar.

4. Process according to Claims 1 to 3, characterised in that 2 to 10 mols of hydrogen fluoride, relative to 1 mol of the alkylbenzotrifluoride, are employed.

**Revendications**

1. Procédé pour isomériser les benzotrifluorures substitués par un groupe alkyle et éventuellement par un reste de fluor, de chlore ou de brome, caractérisé en ce que l'on traite un mélange d'isomères des alkylbenzotrifluorures par le fluorure d'hydrogène.

2. Procédé selon la revendication 1, caractérisé en ce que l'on effectue la réaction dans l'intervalle de température de 0 à 200 °C.

3. Procédé selon les revendications 1 et 2, caractérisé en ce que le procédé est mis en œuvre dans l'intervalle de pression de 1 à 150 bars.

4. Procédé selon les revendications 1 à 3, caractérisé en ce que l'on utilise de 2 à 10 moles de fluorure d'hydrogène pour 1 mole de l'alkylbenzotrifluorure.